# EUROPEAN PATENT SPECIFICATION

(11) **EP 2 277 048 B1**
(45) Date of publication and mention of the grant of the patent: **27.03.2013**
(21) Application number: 09727083.9
(22) Date of filing: 31.03.2009
(51) Int. Cl.: G01N 33/543

(54) **A METHOD FOR SENSING A CHEMICAL**
VERFAHREN ZUR WAHRNEHMUNG EINER CHEMIKALIE
PROCÉDÉ DE DÉTECTION D'UNE SUBSTANCE CHIMIQUE

(30) Priority: 02.04.2008 GB 0805951; 02.04.2008 US 41830 P; 16.09.2008 GB 0816926
(43) Date of publication of application: 26.01.2011
(73) Proprietor: Vivacta Limited, Kent Science Park Sittingbourne Kent ME9 8GU (GB)
(72) Inventor: CARTER, Timothy, Joseph, Nicholas, Sittingbourne Kent ME9 8GU (GB); ROSS, Steven, Andrew, Sittingbourne Kent ME9 8GU (GB)
(74) Representative: Gillard, Richard Edward
(86) International application number: PCT/GB2009/050311
(87) International publication number: WO 2009/122207

(56) References cited:
- WO-A-2004/090512
- WU ET AL: "A selected history and future of immunoassay development and applications in clinical chemistry" CLINICA CHIMICA ACTA, ELSEVIER BV, AMSTERDAM, NL, vol. 369, no. 2, 31 July 2006 (2006-07-31), pages 119-124, XP025058878 ISSN: 0009-8981 [retrieved on 2006-07-31]
- N. KOBAYASHI AND J. GOTO: "Noncompetitive immunoassays for small molecules with high sensitivity and specificity" 2001, ACADEMIC PRESS, EDS. H.E. SPIEGEL , XP008108308 cited in the application page 139 - page 170

## Description

The present invention relates to a method for sensing a chemical, and in particular to an immunoassay employing a chemical sensing device containing a piezo/pyroelectric transducer.

An immunoassay is a test which measures the presence or more usually the concentration of an analyte in a biological fluid. It typically involves the specific binding of an antigen to an antibody. The antibody can be polyclonal or monoclonal, monoclonal antibodies having several benefits, including reproducibility of manufacture and containment of binding to one epitope of an analyte. In order to provide a quantifiable measure of the concentration of the analyte, the response is compared to standard samples of known concentration. The concentration of the antibody or antigen may be determined by a variety of methods, although one of the most common is to label either the antigen or antibody and detect the presence of the label.

Immunoassays can be competitive or non-competitive. In a competitive immunoassay, the antigen in the unknown sample competes with labelled antigen (the "reporter") to bind to antibodies, which are typically immobilised on a solid phase. The amount of labelled antigen bound to the antibody site is then measured, usually by separating and measuring the labelled antigen bound to the solid phase. Clearly the response will be inversely proportional to the concentration of antigen in the unknown sample. In an analogous assay principle, labelled antibody in solution competes with antigen immobilised on a solid phase and that present in the sample, giving a similar inverse proportionality. In a non-competitive immunoassay, also referred to as an immunometric assay, the antigen in the unknown sample binds to an excess of immobilised antibodies (the "capture" antibodies) and the amount of bound antigen is measured. Unlike the competitive method, the results of the non-competitive method will be directly proportional to the concentration of the antigen. In a so-called "two-site" immunometric assay, also termed a "sandwich assay", the antigen is bound to the capture antibody site, and then labelled antibody is introduced which binds to the antigen bound to the capture antibody. The amount of labelled antibody at the site is then measured.

In a typical sandwich immunoassay, an antibody specific for an antigen of interest is attached to a polymeric support such as a sheet of polystyrene. A drop of the sample to be tested, e.g. a cell extract or a sample of serum or urine, is laid on the sheet, which is washed after formation of the antibody-antigen complex. Antibody specific for a different site on the antigen is then added, and the support is again washed. This second antibody carries a label (the labelled reporter) so that it can be detected with high sensitivity. The amount of second antibody bound to the sheet is proportional to the quantity of antigen in the sample. This assay and other variations on this type of assay are well known, see, for example, "The Immunoassay Handbook, 2nd Ed." David Wild, Ed., Nature Publishing Group, 2001.

Immunoassays of this type work particularly well for large molecular mass analytes, principally because two or more epitopes may be addressed; areas of complementarity between analyte and antibody are relatively large and relatively large differences occur between analytes, e.g. by at least an amino acid in peptides. With small molecule (sometimes referred to as a "hapten", being a small molecule which, when attached to a large carrier such as a protein, can elicit an immune response) immunoassays, the absence of two epitopes prohibits the formation of a "sandwich". This has provided motivation for further techniques to be developed.

One relatively new immunoassay technique is the so-called "selective antibody immunometric" assay which is designed to improve specificity and sensitivity of small molecule detection (see, for example, N. Kobayashi and J. Goto, in Advances in Clinical Chemistry, Vol. 36, Ed. H.E. Spiegel et al., Academic Press, 2001, pages 139-170 and particularly section 3.3 at pages 155-158). This immunoassay also has the advantage that it provides a direct relationship between the concentration of the analyte and signal, rather than the inverse relationship commonly seen in competitive immunoassays.

The protocol is based on the masking of unoccupied antibody sites with a multiply hapten-labelled macromolecule to permit subsequent selective determination of hapten-occupied antibodies. Firstly, anti-hapten antibodies are immobilised on (attached to) a solid support. The solid support is then exposed to a sample containing the unknown analyte (hapten) and a macromolecule multiply conjugated to the hapten. The analyte and the macromolecule then compete for the binding sites of the anti-hapten antibodies on the solid support. The analyte can be introduced prior to the macromolecule, or they can be introduced simultaneously and allowed to compete for the surface or, in the case where the macromolecule hinds reversibly to the surface, the macromolecule can be introduced prior to the analyte. A labelled secondary antibody which binds to the primary antibody is then added. The secondary antibody binds to the primary antibodies which are not bound to the macromolecule. Since the proportion of the primary antibody which is not bound to the macromolecule is directly proportional to the amount of analyte, the assay provides a determination of the amount of analyte present in the sample.

This has been shown to be very sensitive and specific but, as currently practised, requires a number of incubation and washing steps, most importantly to eliminate excess unbound label before determination of the amount of labelled antibody present. This significantly adds to the complexity of the assay and substantially limits the applicability of the technique.

WO 2004/090512 discloses a device for detecting energy generated by non-radiative decay generated in a substance on irradiation with electromagnetic radiation. Wu et al., Clinica Chimica Acta, 2006, 369, 119-124 discloses a selected history and future of immunoassay development and applications in clinical chemistry. N. Kubayashi and J. Goto, Academic Press, 2001, 139-170 discloses non-competitive immunoassays for small molecules with high sensitivity and specificity.

Accordingly, the present invention provides a method for detecting an analyte in a sample, comprising the steps of:

A method for detecting an analyte in a sample, comprising the steps of:
(i) providing a transducer comprising a pyroelectric or piezoelectric element and electrodes which is capable of transducing a change in energy to an electrical signal, and a first reagent immobilised on the transducer, wherein the first reagent has a binding site which is capable of binding to a second reagent in the presence of the analyte;
(ii) introducing the second reagent, the second reagent having a binding site which is capable of binding the first reagent in the presence of the analyte and having a label attached thereto which is capable of absorbing electromagnetic radiation to generate energy by non-radiative decay and which is selected from a carbon particle, a coloured-polymer particle, a dye molecule, an enzyme, a fluorescent molecule, a metal particle, a haemoglobin molecule, a magnetic particle, a nanoparticle having a non-conducting core material and at least one metal shell layer, a red blood cell, and combinations thereof, wherein either the first or the second reagent is capable of binding to the analyte;
(iii) exposing the sample to the transducer thereby allowing the analyte to bind either to the first or to the second reagent;
(iv) introducing a masking reagent which is capable of binding to the same first or second reagent as the analyte, thereby preventing binding of the first reagent to the second reagent;
(v) irradiating the sample with electromagnetic radiation;
(vi) transducing the energy generated into an electrical signal; and
(vii) detecting the electrical signal, wherein steps (ii) to (iv) may occur in any order.

Thus, the labelled second reagent (the reporter) can only bind to the first (immobilised) reagent in the presence of the analyte and not in the presence of the masking reagent allowing the analyte to compete with the masking reagent for binding to the first or second reagent. As a result of the use of a transducer having a piezo/pyroelectric film, the benefit of being able to detect the binding of the second (labelled) reagent in real time without separation and washing steps is achieved.

The present invention also provides a
kit comprising (i) a device for detecting an analyte in a sample comprising a transducer having a pyroelectric or piezoelectric element and electrodes which is capable of transducing a change in energy to an electrical signal; a first reagent immobilised on the transducer, the first reagent having a binding site which is capable of binding to a second reagent in the present of the analyte; (ii) a second reagent, the second reagent having a binding site which is capable of binding the first reagent in the presence of the analyte and having a label attached thereto which is capable of absorbing electromagnetic radiation to generate energy by non-radiative decay and which is selected from a carbon particle, a coloured-polymer particle, a dye molecule, an enzyme, a fluorescent molecule, a metal particle, a haemoglobin molecule, a magnetic particle, a nanoparticle having a non-conducting core material and at least one metal shell layer, a red blood cell, and combinations thereof, and (iii) a masking reagent which is capable of binding to the same first or second reagent as the analyte thereby preventing binding of the first reagent to the second reagent.

The present invention will now be described with reference to the drawings, in which:
- Fig. 1: shows a device according to WO 2004/090512;
- Fig. 2: shows a schematic representation of the method of the present invention;
- Fig. 3: shows a device for use in the kit according to the present invention; and
- Fig. 4: shows a graph of counts against time, using the method of the present invention.

The method of the present invention provides for the detection of an analyte in a sample. As a first step, the method includes the provision of a transducer having a pyroelectric or piezoelectric element and electrodes which is capable of transducing a change in energy to an electrical signal and exposing the sample to the transducer. Such transducers are known in the art, see for example WO 90/13017 and WO 2004/090512.

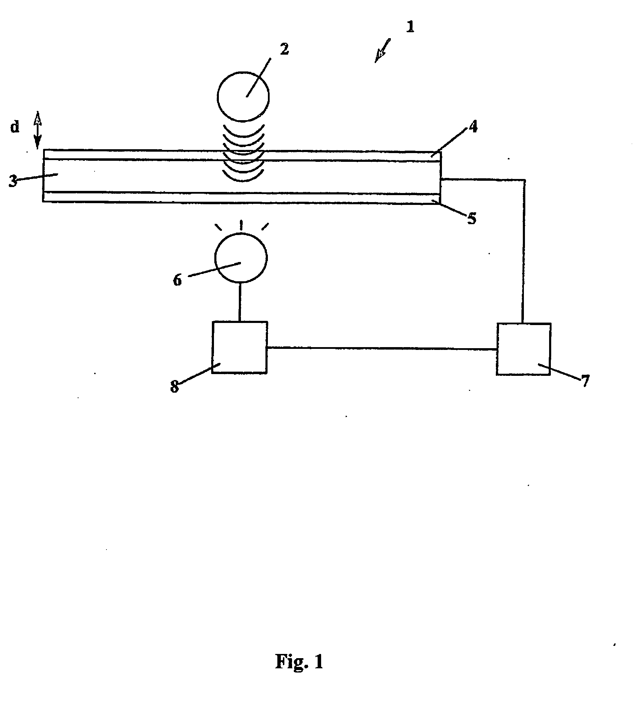

In this regard, Fig. 1 shows the principle of the chemical sensing device 1 suitable for use in the present invention. The device 1 relies on heat generation in a substance 2 on irradiation of the substance 2 with electromagnetic radiation. The device 1 comprises a pyroelectric or piezoelectric transducer 3 having electrode coatings 4,5. The transducer 3 is preferably a film, e.g. a poled polyvinylidene fluoride film. The electrode coatings 4,5 are preferably formed from indium tin oxide having a thickness of about 35 nm, although almost any thickness is possible from a lower limit of 1 nm below which the electrical conductivity is too low and an upper limit of 100 nm above which the optical transmission is too low (it should not be less than 95%T). A substance 2 is held on or proximal to the transducer 3 using any suitable technique, shown here attached to the upper electrode coating 4. The reagent may be in any suitable form and a plurality of reagents may be deposited. Preferably, the substance 2 is adsorbed on to the upper electrode, e.g. covalently coupled or bound via intermolecular forces such as ionic bonds, hydrogen bonding or van der Waal's forces. A key feature of this device is that the substance 2 generates heat when irradiated by a source of electromagnetic radiation 6, such as light, preferably visible light. The light source may be, for example, an LED.

The light source 6 illuminates the substance 2 with light of the appropriate wavelength (e.g. a complementary colour). Although not wishing to be bound by theory, it is believed that the substance 2 absorbs the light to generate an excited state which then undergoes non-radiative decay thereby generating energy, indicated by the curved lines in Fig. 1. This energy is primarily in the form of heat (i.e. thermal motion in the environment) although other forms of energy, e.g. a shock wave, may also be generated.

The energy is, however, detected by the transducer and converted into an electrical signal. The device of the present invention is calibrated for the particular reagent being measured and hence the precise form of the energy generated by the non-radiative decay does not need to be determined. Unless otherwise specified the term "heat" is used herein to mean the energy generated by non-radiative decay. The light source 6 is positioned so as to illuminate the substance 2. Preferably, the light source 6 is positioned substantially perpendicular to the transducer 3 and electrodes 4,5 and the substance 2 is illuminated through the transducer 3 and electrodes 4,5. The light source may be an internal light source within the transducer in which the light source is a guided wave system. The wave guide may be the transducer itself or the wave guide may be an additional layer attached to the transducer. The wavelength of illumination depends on the label used; for example, for 40nm gold labels the preferred wavelength is 525 nm and for carbon labels the preferred wavelength is 650 nm.

The energy generated by the substance 2 is detected by the transducer 3 and converted into an electrical signal. The electrical signal is detected by a detector 7. The light source 6 and the detector 7 are both under the control of the controller 8.

In one embodiment, the light source 6 generates a series of pulses of light (the term "light" used herein means any form of electromagnetic radiation unless a specific wavelength is mentioned) which is termed "chopped light". In principle, a single flash of light, i.e. one pulse of electromagnetic radiation, would suffice to generate a signal from the transducer 3. However, in order to obtain a reproducible signal, a plurality of flashes of light are used which in practice requires chopped light. The frequency at which the pulses of electromagnetic radiation are applied may be varied. At the lower limit, the time delay between the pulses must be sufficient for the time delay between each pulse and the generation of an electrical signal to be determined. At the upper limit, the time delay between each pulse must not be so large that the period taken to record the data becomes unreasonably extended. Preferably, the frequency of the pulses is from 2-50 Hz, more preferably 5-15 Hz and most preferably 10 Hz. This corresponds to a time delay between pulses of 20-500 ms, 66-200 ms and 100 ms, respectively. In addition, the so-called "mark-space" ratio, i.e. the ratio of on signal to off signal is preferably one although other ratios may be used to advantage in certain situations. Sources of electromagnetic radiation which produce chopped light with different frequencies of chopping or different mark-space ratios are known in the art. The detector 7 determines the time delay (or "correlation delay") between each pulse of light from light source 6 and the corresponding electrical signal detected by detector 7 from transducer 3. The applicant has found that this time delay is a function of the distance, d.

Any method for determining the time delay between each pulse of light and the corresponding electrical signal which provides reproducible results may be used. Preferably, the time delay is measured from the start of each pulse of light to the point at which a maximum in the electrical signal corresponding to the absorption of heat is detected as by detector 7.

Thus substance 2 may be separated from the transducer surface and a signal may still be detected. Moreover, not only is the signal detectable through an intervening medium capable of transmitting energy to the transducer 3, but different distances, d, may be distinguished (this has been termed "depth profiling") and that the intensity of the signal received is proportional to the concentration of the substance 2 at the particular distance, d, from the surface of the transducer 3.

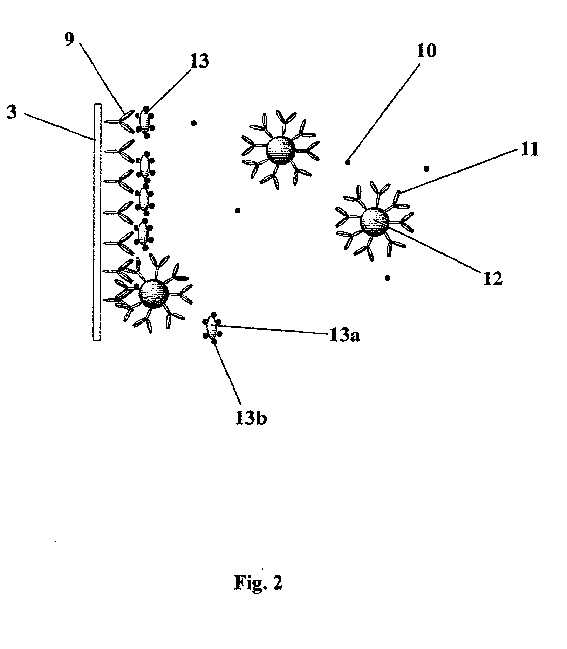

Fig. 2 shows the incorporation of the device 1 from Fig. 1 in a selective antibody immunometric assay of in accordance with the present invention. The transducer 3 is shown in a vertical arrangement, although other orientations are possible and even advantageous in some circumstances. The transducer 3 is coated with a first reagent shown in Fig. 2 as a first antibody 9 (an immobilised capture antibody). The sample also contains an analyte 10 and a second antibody 11 bound to a label 12 (which corresponds to the substance 2 in Fig. 1).

The first antibody 9 has been raised against the analyte 10 and selectively binds to the analyte 10 when the sample is introduced. However, a masking reagent 13 is also incorporated into the sample. In this example; the masking reagent 13 is capable of binding to the first antibody 9. The masking reagent 13 is typically a macromolecule 13a having multiple sites 13b which are capable of binding to the first reagent 9. The binding sites on the macromolecule are preferably moieties having the same chemical structure as the analyte

The macromolecule may be any molecule which provides the necessary steric hindrance to the binding of the second reagent 11 and which may be multiply conjugated. Examples include polysaccharides, synthetic polymers, such as polystyrene or latex, and polypeptides, such as poly(L-lysine). The molecular weight of the macromolecule is preferably 10 times that of the analyte, more preferably 100 to 1000 times that of the analytc, e.g. with a molecular weight of 5,000 to 500,000 Daltons. One particular type of masking agent which can be used is an idiotypic antibody, which specifically recognises the binding site of the capture agent on the surface.

Fig. 2 shows the masking reagent 13 bound to a number of the first antibodies 9 on the surface of the transducer 3. However, two of the first antibodies 9 are shown bound to the analyte 10. The antibodies 9 bound to the analyte 10 are capable of further binding to the second antibody 11 attached to the label 12. The second reagent 11 has a binding site which is capable of binding the first reagent 9 in the presence of the analyte 10 but not in the presence of the masking reagent 13 since the masking reagent 13 blocks the binding site, principally by steric hindrance.

Importantly, the method of the present invention permits detection of the binding of the second antibody 11 to the first reagent 9 in real time, without separation and washing steps. This is a significant advantage in the art. Thus, in a preferred embodiment, the assay is carried out without removing the sample from the transducer 3 between the steps of exposing the sample to the transducer 3 and irradiating the sample. Moreover, no further intervention (e.g. to separate bound and unbound second reagent) is required during steps (ii) to (iv).

The second reagent which is not bound to the surface is free to diffuse away from the surface. Preferably the second reagent is allowed to become separated from the surface solely by diffusion.

Fig. 2 shows an embodiment where the masking reagent binds to the first reagent, although other examples are included within the scope of the present invention. A key feature of the present invention is that the transducer is exposed to the sample in the presence of the masking reagent and that the masking agent is capable of binding to either the first or the second reagent to prevent binding of the first reagent to the second reagent. The masking reagent must also bind to the same first or second reagent as the analyte. Thus, if the first reagent is capable of binding the analyte the first reagent will also bind to the masking reagent, and the second reagent will not bind to analyte or the masking reagent. The same applies to the second reagent, mutatis mutandis. By disrupting this binding and competing with the analyte, the masking reagent is able to cause the amount of the second reagent to be bound to the first reagent to be proportional to the amount of the analyte present in the sample.

In this regard, the masking reagent may be bound releasably to the first or second reagent prior to exposure of the transducer to the sample (the first or second reagent is pre-incubated with the masking reagent), or the masking reagent may be introduced at substantially the same time as the sample.

Where the masking reagent is bound to the first or second reagent prior to exposure of the transducer to the sample, it must be bound releasably to allow the analyte to bind to the first or second reagent. By releasable, binding is meant that the masking reagent is capable of binding to the first or second reagent, but that the masking reagent is displaced by the binding of the analyte to the said first or second reagent. Where the masking reagent is introduced at substantially the same time as the sample, the binding may be releasable, but this is not essential since the masking reagent can still compete with the analyte for binding to the first or second reagent. Where the binding is releasable, preferably the masking reagent binds reversibly to the first or second reagent, i.e. an equilibrium exists between the bound and unbound states.

Although the first and second reagents are exemplified in Fig. 2 by a first and second antibody, the present invention is not limited thereto. Thus, although the first and second reagents are preferably antibodies, other reagents may also be used, such as nucleic acids. In yet preferred embodiment, the present invention provides a method of performing a selective antibody immunometric assay to detect an analyte (sometimes referred to as a "hapten", being a small molecule which, when attached to a large carrier such as a protein, can elicit an immune response) in a sample, comprising the steps of:
(i) providing a transducer comprising a pyroelectric or piezoelectric element and electrodes which is capable of transducing a change in energy to an electrical signal, and a first antibody immobilised on the transducer, wherein the first antibody is capable of binding to a second antibody in the presence of the analyte; (ii) introducing the second antibody which is capable of binding the first antibody in the presence of the analyte and having a label attached thereto which is capable of absorbing electromagnetic radiation to generate energy by non-radiative decay and which is selected from a carbon particle, a coloured-polymer particle, a dye molecule, an enzyme, a fluorescent molecule, a metal particle, a haemoglobin molecule, a magnetic particle, a nanoparticle having a non-conducting core material and at least one metal shell layer, a red blood cell, and combinations thereof, wherein either the first or the second antibody is capable of binding to the analyte; (iii) exposing the sample to the transducer thereby allowing the analyte to bind either to the first or to the second antibody; (iv) introducing a masking reagent which is capable of binding to the same first or second antibody; (v) irradiating the sample with electromagnetic radiation; (vi) transducing the energy generated into an electrical signal; and (vii) detecting the electrical signal, wherein steps (ii) to (iv) may occur in any order. Preferably the binding is reversible.

The first reagent 9 is shown in Fig. 2 attached to the surface of the transducer 3 and is preferably adsorbed on to the transducer. The surface may also be covered by further coatings to stabilise the surface, e.g. Stabilcoat from SurModics Inc, Eden Prairie, MN, USA.

As discussed with reference to Fig. 2, the second reagent 11 has a label 12 attached thereto. The label 12 is capable of absorbing the electromagnetic radiaition generated by the radiation source to generate energy by non-radiative decay. Thus, to detect the presence of the label 12 proximal to the transducer 3, the sample is irradiated with a series of pulses of electromagnetic radiation. The transducer 3 transduces the energy generated into an electrical signal and the electrical signal is detected by detector 7.

The label 12 is capable of interacting with the electromagnetic radition generated by the radiation source to generate energy by non-radiative decay. The label is selected from a carbon particle, a coloured-polymer particle (e.g. couloured latex), a dye molecule, an enzyme, a fluorescent molecule, a metal (e.g. gold) particle, a haemoglobin molecule, a magnetic particle, a nanoparticle having a non-conducting core material and at least one metal shell layer, a red blood cell, and combinations thereof.

In the case of a magnetic particle, the electromagnetic radiation is radio frequency radiation. All of the other labels mentioned hereinabove employ light, which can include IR or UV radiation. Preferably the label is a gold particle or a carbon particle. Carbon particles have benefits in that they absorb essentially uniformly at all wavelengths of interest and are much less dense than most metallic particles minimising their sedimentation during the assay. Gold particles are commercially available or may be prepared using known methods (see for example G. Frens, Nature, 241, 20-22 (1973)). For a more detailed explanation of the nanoparticle label see US 6,344,272 and WO 2007/141581.

Preferably, the present invention uses a particle having a particle size of 20 to 1,000 nm, more preferably 100 to 500 nm. By particle size is meant the diameter of the particle at its widest point.

The label 12 is proximal to the transducer when the binding event has occurred. That is, the label is sufficiently close to the surface of the transducer for the transducer to be able to detect the energy generated by the label on irradiation of the sample. The actual distance between the label and the surface of the transducer will, however, depend on a number of variables, such as the size and nature of the label, the size and nature of the first and second antibodies and the analyte, the nature of the sample medium, and the nature of the electromagnetic radiation and the corresponding setting of the detector. With regard to the nature of the electromagnetic radiation, the device of the present invention may include a radiation source which is adapted to generate a series of pulses of electromagnetic radiation and the detector is adapted to determine the time delay between each pulse of electromagnetic radiation from the radiation source and the generation of the electric signal thereby allowing a precise determination of the position of the label with respect to the transducer as discussed with reference to Fig. 1.

The unknown sample is expected to contain the analyte, but of course the assay of the present invention may be used to determine the presence or absence of the analyte. The analyte is preferably a small molecule insofar as the assay is ideally suited for such a molecule, although the present invention is not limited thereto. The term "small molecule" used herein is a term of the art and is used to distinguish the molecule from macromolecules such as proteins and nucleic acids. A small molecule is often referred to in the field of immunoassays as a "hapten", being a small molecule which, when attached to a large carrier such as a protein can elicit an immune response and includes molecules such as hormones and synthetic drugs. A small molecule of this type will typically have a molecular weight of 2,000 or less, often 1,000 or less and even 500 or less. The first reagent may be adapted to bind to the analyte itself, although the analyte can undergo a chemical reaction or initial complexing event before binding to the first reagent. For example, the analyte might be protonated/deprotonated in the pH of the assay conditions.

The sample which may or may not contain the analyte of interest will generally be a fluid sample and usually a biological sample, such as a bodily fluid (hence aqueous), e.g. blood, plasma, saliva, serum or urine. The sample may contain suspended particles and may even be whole blood. An advantage of the method of the present invention is that the assay may be performed on a sample which does contain suspended particles without unduly influencing the results of the assay. The sample will typically be in the order of microlitres (e.g. 1-100 µL. preferably 1-10 µL). In order to hold a fluid sample, the transducer is preferably located in a sample chamber and more preferably a well. In a preferred embodiment, the transducer is integral with the chamber, i.e. it forms one of the walls which define the chamber. The sample may simply be retained by surface tension forces, for example, inside a capillary channel.

The present invention also provides a kit for performing the assay described herein. The kit comprises a device for detecting an analyte in a sample substantially as described herein with reference to Fig. 1. The device comprises a transducer having a pyroelectric or piezoelectric element and electrodes which is capable of transducing a change in energy to an electrical signal, a first reagent immobilised on the transducer, the first reagent having a binding site which is capable of binding the analyte masking reagent, either added to the sample containing the analyte or bound releasably to the first reagent prior to the addition of the sample, a source of electromagnetic radiation, and a detector for detecting the electrical signal. The kit further comprises the second reagent, the second reagent having a binding site which is capable of binding the first reagent in the presence of the analyte but not in the presence of the masking reagent. The second reagent may be dried down on to one of the interior surfaces of the chamber prior to use so that it is released when the sample is introduced. In a preferred embodiment, the device consists essentially of the above-described features. By "essentially" is meant that no other features are required to perform the assay.

The device may take the form of a hand-held portable reader and a disposable device containing the transducer. The sample is collected in an essentially closed system, mixed with the second reagent and placed in a reader that would perform the irradiation of the sample and detection of the resultant electrical signal.

### Examples

### Example 1

### Preparation of active piezo/pyrofilm biosensors

A poled piezoelectric polyvinylidene fluoride (PVDF) bimorph film, coated in indium tin oxide used as the sensing device in the following example, was dip-coated in polystyrene solution (1% in toluene) in a low humidity environment to give a polystyrene layer on top of the indium tin oxide. This was then coated in polystreptavidin solution (200 µg/mL in PBS - 10 mmol/L phosphate buffer, pH 7.5, containing 2.7 mmol/L KCl, 137 mmol/L NaCl and 0.05% Tween) by incubation at room temperature overnight. Polystreptavidin was prepared as described by Tischer et al (US 5,061,640).

To prepare a "capture" surface the polystreptavidin surface was incubated with biotinylated anti-testosterone (M1), giving an antibody coated surface (C1). 10 µg/mL of biotinylated anti-testosterone (HyTest Ltd, Turku, Finland, Cat # 2T2-biotin, or Accurate Chemical Co, Westbury, New York, USA, Cat # BHS113) in PBS was incubated at room temperature overnight and then washed with excess PBS and coated with Stabilcoat (SurModics Inc, Eden Prairie, MN, USA) before drying at 40°C.

Antibodies were biotinylated by methods know to those skilled in the art. For example, a 5mg/ml antibody solution in PBS is prepared by dissolving lyophilised antibody, or by dilution. If this solution contains other proteins or Tris or other interfering agents, purify by dialysis or gel filtration. Then prepare an NHS-biotin solution at 20 mmol/L in anhydrous DMSO and add 15 µL of the solution of NHS-biotin to the antibody (1 mL). Incubate for 1 hour at room temperature and then dialyse the antibody against PBS containing sodium azide (0.01%). The biotinylated antibody can be diluted to 1mg/mL with 0.1 % sodium azide and 20% of glycerol for storage at -20°C or +4°C. The level of biotinylation should be in the range of 1-3 biotins per IgG. This can be estimated by quantitation of biotins or for high biotinylation rates, by a differential quantitation of amines.

### Example 2

### Preparation of the reporter conjugates

Carbon-labelled reporter conjugates were prepared essentially as described by Van Doom et al. (US 5,641,689). To prepare antibody coated reporter conjugates (C2), 1 mL of Special Black-4 RCC nominally 150 nm carbon particles (Degussa, Essen, Germany) in 5 mmol/L phosphate buffer, pH 6.2 was incubated with 200 µg/mL polystreptavidin solution overnight at room temperature with shaking, resulting in a streptavidin-coated surface. The resultant carbon conjugate was washed (by centrifugation, pelleting and resuspension). 10 µg/mL of biotinylated secondary monoclonal antibodies (M2), reactive against the antibody species used as a source for the anti-testosterone capture antibody (M1) in PBS was then incubated overnight with 1 mL of this streptavidin-coated carbon particle suspension with shaking. The resultant carbon conjugate (C2) was washed (by centrifugation, pelleting and resuspension) 3 times with 0.05 mol/L borate buffer at pH 8.5 and stored in this buffer in the dark at 4°C. For clarity, if the anti-testosterone antibody (M1) was a monoclonal mouse antibody, the secondary monoclonal antibody (M2) was a goat antibody raised to react against whole intact mouse antibody (so called "goat anti-mouse antibody"). Of course, any other species pairs, both mono and poly-clonal, could be used and would be well-known to those skilled in the art.

### Example 3

### Preparation of masking reagent

To prepare the antigen coated masking reagent (MR1), 1 mL of streptavidin-coated polystyrene latex particles (nominal diameter 23 nm), Bangs Laboratories P/N PS02N/8192 (Bangs Laboratories Inc, Fishers IN, USA) in 5 mmol/L phosphate buffer, pH 6.2 was incubated with 30 nmol/L of 7α-C6-biotinylated testosterone, prepared as described in Luppa et al. (Clin. Chem. 1997, 43, 2345, at room temperature overnight with shaking. The resultant conjugate was washed 3 times with 0.05mol/L borate buffer at pH 8.5 as above and stored in this buffer in the dark at 4°C.

### Example 4

### Preparation of masked antibody coated film

To prepared a masked piezofilm surface (C3), a piece of antibody-coated piezofilm (C1, described above) was incubated with antigen-coated masking reagent (MR1 above) in PBS at room temperature overnight and then washed with excess PBS and coated with Stabilcoat (SurModics Inc, Eden Prairie, MN, USA) before drying at 40°C.

### Example 5

### Assay - Antibody-coated piezo/pyrofilm sensor and masking reagent

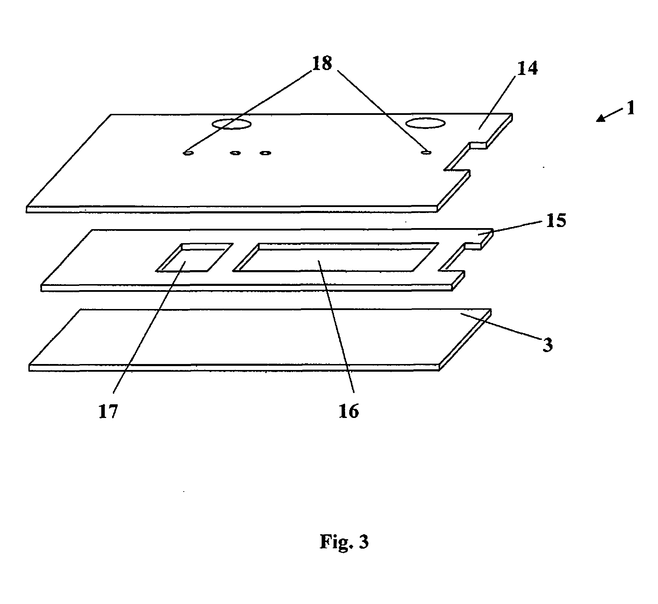

As shown in Fig. 3, a sensor 1 was fabricated to perform the assay. The sensor 1 is fabricated from a piece of antibody-coated piezofilm 3 (C3, described hereinabove) and a piece of transparent polycarbonate lidding film 14. The films are spaced at a distance of approximately 500 microns using a spacer 15 composed of a piece of pressure sensitive adhesive-coated polyester film die-cut to form two unequally sized chambers 16,17; one chamber 16 of approximate dimensions 30 x 10 x 0.5 mm for the assay reaction and a second smaller chamber 17 of dimensions 10 x 10 x 0.5 mm for a control reaction. Provision is made to allow for electrical connections to the top and bottom surfaces of the piezofilm in order to detect the charge generated.

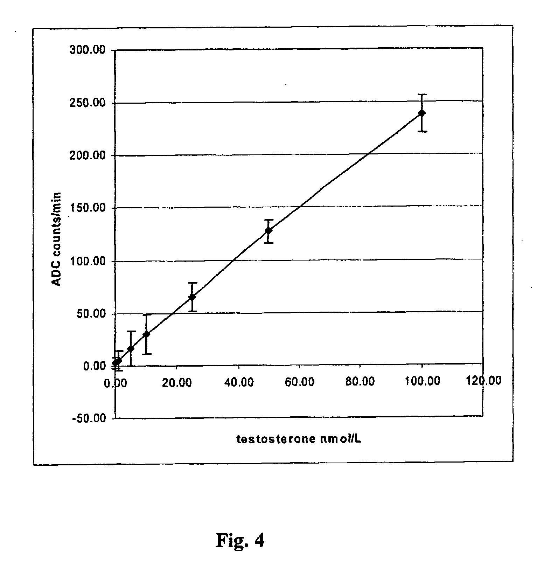

Assays are carried out by filling the larger chamber 16 (through a fill hole 18) with a mixture of 0.1 mol/L Tris buffer, containing 0.150 mol/L MgCl₂ and 0.075% Tween 20 solution, containing 150 nm colloidal carbon particles (at a final concentration of 0.0025% solids) coated with biotinylated antibody (C2, as described hereinabove), reactive against the capture antibody (M1), and testosterone standards in PBS to give a final concentration range of 0.1-100 nmol/L. The control chamber 17 is simultaneously filled with an identical reaction mix to that in the assay chamber with the testosterone standard replaced with PBS. The entry and exit holes are sealed and the chamber assembly is connected to a test instrument such that the piezofilm 3 is oriented vertically on the side face of the chamber. The piezofilm is then illuminated with chopped LED light sequentially with four LEDs (of wavelength 625 nm), of which three illuminate different areas of the surface of the assay chamber and one illuminates the piezofilm surface of the control chamber. For each LED pulse, a voltage is measured across the piezofilm using a lock-in amplifier and analogue to digital (ADC) converter. The ADC signal is plotted over time and the relationship of ADC counts/min against testosterone concentration is shown in Fig. 4.

## Claims

1. A method for detecting an analyte in a sample, comprising the steps of:
(i) providing a transducer comprising a pyroelectric or piezoelectric element and electrodes which is capable of transducing a change in energy to an electrical signal, and a first reagent immobilised on the transducer, wherein the first reagent has a binding site which is capable of binding to a second reagent in the presence of the analyte;
(ii) introducing the second reagent, the second reagent having a binding site which is capable of binding the first reagent in the presence of the analyte and having a label attached thereto which is capable of absorbing electromagnetic radiation to generate energy by non-radiative decay and which is selected from a carbon particle, a coloured-polymer particle, a dye molecule, an enzyme, a fluorescent molecule, a metal particle, a haemoglobin molecule, a magnetic particle, a nanoparticle having a non-conducting core material and at least one metal shell layer, a red blood cell, and combinations thereof, wherein either the first or the second reagent is capable of binding to the analyte;
(iii) exposing the sample to the transducer thereby allowing the analyte to bind either to the first or to the second reagent;
(iv) introducing a masking reagent which is capable of binding to the same first or second reagent as the analyte thereby preventing binding of the first reagent to the second reagent;
(v) irradiating the sample with electromagnetic radiation;
(vi) transducing the energy generated into an electrical signal; and
(vii) detecting the electrical signal, wherein steps (ii) to (iv) may occur in any order.

2. A method as claimed in claim 1, wherein the first and second reagents are antibodies.

3. A method as claims in claims 1 or 2, wherein the masking reagent is introduced at substantially the same time as the sample.

4. A method as claimed in any preceding claim, wherein the transducer is located in a sample chamber.

5. A method as claimed in any preceding claim, wherein the sample contains suspended particles.

6. A method as claimed in claim 5, wherein the sample is whole blood.

7. A method as claimed in any preceding claim, wherein the radiation source is adapted to generate a series of pulses of electromagnetic radiation and the detector is adapted to determine the time delay between each pulse of electromagnetic radiation from the radiation source and the generation of the electrical signal.

8. A method as claimed in any preceding claim, wherein the method is carried out without removing the sample from the transducer between the steps of exposing the sample to the transducer and irradiating the sample.

9. A kit comprising (i) a device for detecting an analyte in a sample comprising a transducer having a pyroelectric or piezoelectric element and electrodes which is capable of transducing a change in energy to an electrical signal; a first reagent immobilised on the transducer, the first reagent having a binding site which is capable of binding to a second reagent in the presence of the analyte; (ii) a second reagent, the second reagent having a binding site which is capable of binding the first reagent in the presence of the analyte and having a label attached thereto which is capable of absorbing electromagnetic radiation to generate energy by non-radiative decay and which is selected from a carbon particle, a coloured-polymer particle, a dye molecule, an enzyme, a fluorescent molecule, a metal particle, a haemoglobin molecule, a magnetic particle, a nanoparticle having a non-conducting core material and at least one metal shell layer, a red blood cell, and combinations thereof, and (iii) a masking reagent which is capable of binding to the same first or second reagent as the analyte, thereby preventing binding of the first reagent to the second reagent.

10. A kit as claimed in claim 9, wherein the first and second reagents are antibodies.

11. A kit as claimed in claims 9 or 10, wherein the device further comprises a sample chamber and the transducer is located in the sample chamber.

12. A kit as claimed in Claim 11, wherein the transducer is integral with the chamber.

13. A kit as claimed in any of claims 9 to 12, wherein the radiation source is adapted to generate a series of pulses of electromagnetic radiation and the detector is adapted to determine the time delay between each pulse of electromagnetic radiation from the radiation source and the generation of the electrical signal.

## Patentansprüche

1. Verfahren zum Nachweisen eines Analyts in einer Probe, das die folgenden Schritte beinhaltet:
(i) Bereitstellen eines Wandlers, der ein pyroelektrisches oder piezoelektrisches Element sowie Elektroden umfasst, die eine Energieänderung in ein elektrisches Signal umwandeln können, und eines auf dem Wandler immobilisierten ersten Reagenzes, wobei das erste Reagenz eine Bindungsstelle hat, die sich in Anwesenheit des Analyts an ein zweites Reagenz binden kann;
(ii) Einleiten des zweiten Reagenzes, wobei das zweite Reagenz eine Bindungsstelle hat, die das erste Reagenz in Anwesenheit des Analyts binden kann, und eine Markierung daran angebracht hat, die elektromagnetische Strahlung absorbieren kann, um Energie durch nichtstrahlenden Zerfall zu erzeugen, und das ausgewählt ist aus einem Kohlenstoffpartikel, einem gefärbten Polymerpartikel, einem Farbstoffmolekül, einem Enzym, einem fluoreszenten Molekül, einem Metallpartikel, einem Hämoglobinmolekül, einem magnetischen Partikel, einem Nanopartikel mit einem nichtleitenden Kernmaterial und wenigstens einer Metallmantelschicht, einem roten Blutkörperchen und Kombinationen davon, wobei sich entweder das erste oder das zweite Reagenz an den Analyt binden kann;
(iii) Inkontaktbringen der Probe mit dem Wandler, um es zuzulassen, dass sich der Analyt entweder an das erste oder das zweite Reagenz bindet;
(iv) Einleiten eines Markierungsreagenzes, das sich an dasselbe erste oder zweite Reagenz binden kann wie der Analyt, um dadurch eine Bindung des ersten Reagenz an das zweite Reagenz zu verhindern;
(v) Bestrahlen der Probe mit elektomagnetischer Strahlung;
(vi) Umwandeln der erzeugten Energie in ein elektrisches Signal ; und
(vii) Detektieren des elektrischen Signals, wobei die Schritte (ii) bis (iv) in jeder beliebigen Reihenfolge erfolgen können.

2. Verfahren nach Anspruch 1, wobei das erste und das zweite Reagenz Antikörper sind.

3. Verfahren nach Anspruch 1 oder 2, wobei das Maskierungsreagenz im Wesentlichen zur selben Zeit eingeleitet wird wie die Probe.

4. Verfahren nach einem der vorherigen Ansprüche, wobei sich der Wandler in einer Probenkammer befindet.

5. Verfahren nach einem der vorherigen Ansprüche, wobei die Probe suspendierte Partikel enthält.

6. Verfahren nach Anspruch 5, wobei die Probe Vollblut ist.

7. Verfahren nach einem der vorherigen Ansprüche, wobei die Strahlungsquelle so ausgelegt ist, dass sie eine Reihe von Impulsen von elektromagnetischer Strahlung erzeugt, und der Detektor so ausgelegt ist, dass er die Zeitverzögerung zwischen jedem elektromagnetischen Strahlungsimpuls und der Strahlungsquelle und die Erzeugung des elektrischen Signals ermittelt.

8. Verfahren nach einem der vorherigen Ansprüche, wobei das Verfahren ohne Entfernen der Probe vom Wandler zwischen den Schritten des Inkontaktbringens der Probe mit dem Wandler und des Bestrahlens der Probe durchgeführt wird.

9. Kit, der Folgendes umfasst: (i) eine Vorrichtung zum Nachweisen eines Analyts in einer Probe, die einen Wandler mit einem pyroelektrischen oder piezoelektrischen Element und Elektroden umfasst, der eine Energieänderung in ein elektrisches Signal umwandeln kann; ein erstes auf dem Wandler immobilisiertes Reagenz, wobei das erste Reagenz eine Bindungsstelle hat, die sich in Anwesenheit des Analyts an ein zweites Reagenz binden kann; (ii) ein zweites Reagenz, wobei das zweite Reagenz eine Bindungsstelle hat, die in Anwesenheit des Analyts das erste Reagenz binden kann, und an dem eine Markierung angebracht ist, die elektromagnetische Strahlung absorbieren kann, um Energie durch nichtstrahlende Zersetzung zu erzeugen, und das ausgewählt ist aus einem Kohlenstoffpartikel, einem gefärbten Polymerpartikel, einem Farbstoffmolekül, einem Enzym, einem fluoreszenten Molekül, einem Metallpartikel, einem Hämoglobinmolekül, einem magnetischen Partikel, einem Nanopartikel mit einem nichtleitenden Kernmaterial und wenigstens einer Metallmantelschicht, einem roten Blutkörperchen und Kombinationen davon und (iii) ein Maskierungsreagenz, das sich an dasselbe erste oder zweite Reagenz binden kann wie der Analyt, um dadurch eine Bindung des ersten Reagenz an das zweite Reagenz zu verhindern.

10. Kit nach Anspruch 9, wobei das erste und das zweite Reagenz Antikörper sind.

11. Kit nach Anspruch 9 oder 10, wobei die Vorrichtung ferner eine Probenkammer umfasst und der Wandler sich in der Probenkammer befindet.

12. Kit nach Anspruch 11, wobei der Wandler mit der Kammer integriert ist.

13. Kit nach einem der Ansprüche 9 bis 12, wobei die Strahlungsquelle so ausgelegt ist, dass sie eine Reihe von elektromagnetischen Strahlungsimpulsen erzeugt, und der Detektor so ausgelegt ist, dass er die Zeitverzögerung zwischen jedem elektromagnetischen Strahlungsimpuls und der Strahlungsquelle und die Erzeugung des elektrischen Signals ermittelt.

## Revendications

1. Procédé pour détecter une substance à analyser dans un échantillon, comprenant les étapes consistant à :
(i) fournir un transducteur comprenant un élément pyroélectrique ou piézoélectrique et des électrodes, qui est capable de convertir un changement d'énergie en un signal électrique, et un premier réactif immobilisé sur le transducteur, dans lequel le premier réactif a un site de liaison qui est capable de se lier à un deuxième réactif en présence de la substance à analyser ;
(ii) introduire le deuxième réactif, le deuxième réactif ayant un site de liaison qui est capable de lier le premier réactif en présence de la substance à analyser et auquel est fixé une étiquette, qui est capable d'absorber le rayonnement électromagnétique pour générer de l'énergie par décroissance non radiative et qui est sélectionné entre une particule de carbone, une particule de polymère coloré, une molécule colorante, un enzyme, une molécule fluorescente, une particule métallique, une molécule d'hémoglobine, une particule magnétique, une nanoparticule ayant un matériau de noyau non conducteur et au moins une couche d'enveloppe métallique, et un érythrocyte, et des combinaison de ces éléments ;
(iii) exposer l'échantillon au transducteur, en permettant ainsi à la substance à analyser de se lier soit au premier soit au deuxième réactif ;
(iv) introduire un réactif de masquage qui est capable de se lier au même premier ou deuxième réactif que la substance à analyser, en empêchant ainsi la liaison du premier réactif au deuxième réactif ;
(v) l'irradiation de l'échantillon avec un rayonnement électromagnétique ;
(vi) la transduction de l'énergie générée en un signal électrique ; et
(vii) la détection du signal électrique, les étapes (ii) à
(iv) pouvant avoir lieu dans n'importe quel ordre.

2. Procédé selon la revendication 1, dans lequel lesdits premier et deuxième réactifs sont des anticorps.

3. Procédé selon les revendications 1 ou 2, dans lequel le réactif de masquage est introduit essentiellement en même temps que l'échantillon.

4. Procédé selon l'une quelconque des revendications précédentes, dans lequel le transducteur est situé dans une chambre d'échantillon.

5. Procédé selon l'une quelconque des revendications précédentes, dans lequel l'échantillon contient des particules en suspension.

6. Procédé selon la revendication 5, dans lequel l'échantillon est du sang total.

7. Procédé selon l'une quelconque des revendications précédentes, dans lequel la source de rayonnement est adaptée pour générer une série d'impulsions de rayonnement électromagnétique et le détecteur est adapté pour déterminer le délai entre chaque impulsion de rayonnement électromagnétique de la source de rayonnement et la génération du signal électrique.

8. Procédé selon l'une quelconque des revendications précédentes, le procédé étant exécuté sans enlèvement de l'échantillon du transducteur entre les étapes d'exposition de l'échantillon au transducteur et d'irradiation de l'échantillon.

9. Ensemble comprenant (i) un dispositif pour détecter une substance à analyser dans un échantillon comprenant un transducteur ayant un élément pyroélectrique ou piézoélectrique et des électrodes, qui est capable de convertir un changement d'énergie en un signal électrique ; un premier réactif immobilisé sur le transducteur, le premier réactif ayant un site de liaison qui est capable de se lier à un deuxième réactif en présence de la substance à analyser ; (ii) un deuxième réactif, le deuxième réactif ayant un site de liaison qui est capable de lier le premier réactif en présence de la substance à analyser et auquel est fixé une étiquette, qui est capable d'absorber le rayonnement électromagnétique pour générer de l'énergie par décroissance non radiative et qui est sélectionné entre une particule de carbone, une particule de polymère coloré, une molécule colorante, un enzyme, une molécule fluorescente, une particule métallique, une molécule d'hémoglobine, une particule magnétique, une nanoparticule ayant un matériau de noyau non conducteur et au moins une couche d'enveloppe métallique, et un érythrocyte, et des combinaison de ces éléments ; et (iii) un réactif masquant qui est capable de se lier au même premier ou deuxième réactif que la substance à analyser, en empêchant ainsi la liaison du premier réactif au deuxième réactif.

10. Ensemble selon la revendication 9, dans lequel les premier et deuxième réactifs sont des anticorps.

11. Ensemble selon les revendications 9 ou 10, dans lequel le dispositif comprend en outre une chambre d'échantillon et le transducteur est situé dans la chambre d'échantillon.

12. Ensemble selon la revendication 11, dans lequel le transducteur est intégré à la chambre.

13. Ensemble selon l'une quelconque des revendications 9 à 12, dans lequel la source de rayonnement est adaptée pour générer une série d'impulsions de rayonnement électromagnétique et le détecteur est adapté pour déterminer le délai entre chaque impulsion de rayonnement électromagnétique de la source de rayonnement et la génération du signal électrique.
